# EUROPEAN PATENT SPECIFICATION

(11) **EP 4 456 863 B1**
(45) Date of publication and mention of the grant of the patent: **25.06.2025**
(21) Application number: 22830795.5
(22) Date of filing: 07.12.2022
(51) Int. Cl.: A61K 8/365, A61K 8/44, A61Q 19/02

(54) **PERSONAL CARE COMPOSITION**
KÖRPERPFLEGEZUSAMMENSETZUNG
COMPOSITION POUR SOINS PERSONNELS

(30) Priority: 31.12.2021 WO PCT/CN2021/143514; 27.01.2022 EP 22153546
(43) Date of publication of application: 06.11.2024
(73) Proprietor: Unilever IP Holdings B.V., 3013 AL Rotterdam (NL); Unilever Global IP Limited, Wirral, Merseyside CH62 4ZD (GB)
(72) Inventor: GU, Xuelan, 6708 WH Wageningen (NL); MI, Tingyan, 6708 WH Wageningen (NL)
(74) Representative: James, Helen Sarah
(86) International application number: PCT/EP2022/084705
(87) International publication number: WO 2023/126136

(56) References cited:
- WO-A1-2012/072951
- WO-A1-2017/108438
- US-A- 6 153 649
- US-A1- 2020 009 034

## Description

### Field of the Invention

The present invention relates to a personal care composition comprising a carboxymethyl cysteine compound and hydroxyl substituted fatty acid for providing enhanced skin brightening efficacy.

### Background of the Invention

Many consumers are concerned with the characteristics of their skin. For example, consumers are concerned with the degree of pigmentation of their skin, freckles and/or age spots. Other consumers wish to reduce skin darkening caused by exposure to sunlight. To meet the needs of consumers, many attempts have been made to develop products that improve skin characteristics. The products developed thus far, however, often tend to have low efficacy.

US 2020/009034 discloses topical skin lightening compositions comprising hydroxystearic acid as skin lightening active.

The present inventors have acknowledged that there is an increasing interest to develop a personal care composition having high skin brightening efficacy and therefore develop a personal care composition comprising carboxymethyl cysteine compound and hydroxyl substituted fatty acid. It was surprisingly found that such composition is capable of providing an improved skin brightening efficacy.

### Summary of the Invention

In a first aspect, the present invention is directed to a personal care composition comprising carboxymethyl cysteine compound and hydroxyl substituted fatty acid
In a second aspect, the present invention is directed to a method of improving the appearance of skin; reducing pigmentation, age spots and/or discoloration of skin; and/or brightening skin comprising a step of topically applying to the skin the composition of the present invention.

In a third aspect, the present invention is directed to use of composition of the present invention for improving the appearance of skin; reducing pigmentation, age spots and/or discoloration of skin; and/or brightening skin.

All other aspects of the present invention will more readily become apparent upon considering the detailed description and examples which follow.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

The carboxymethyl cysteine compound refers to compound selected from carboxymethyl cysteine, salt of carboxymethyl cysteine, ester of carboxymethyl cysteine, amide of carboxymethyl cysteine or a mixture thereof. Preferably, the carboxymethyl cysteine compound comprises carboxymethyl cysteine, ester of carboxymethyl cysteine, and/or salt of carboxymethyl cysteine. More preferably, the carboxymethyl cysteine compound comprises carboxymethyl cysteine, and/or salt of carboxymethyl cysteine. Even more preferably, carboxymethyl cysteine compound comprises salt of carboxymethyl cysteine. Still even more preferably the carboxymethyl cysteine compound comprises lysine carboxymethyl cysteinate and most preferably, the carboxymethyl cysteine compound is lysine carboxymethyl cysteinate.

Preferably, the carboxymethyl cysteine compound is present in amount of at least 0.00001%, more preferably at least 0.0001%, even more preferably at least 0.001%, still even more preferably at least 0.01%, and most preferably at least 0.1% by weight of the composition.

Preferably, the carboxymethyl cysteine compound is present in amount of no greater than 10%, more preferably no greater than 5%, even more preferably no greater than 3%, still even more preferably no greater than 1%, and most preferably no greater than 0.5% by weight of the composition.

Preferably, the lysine carboxymethyl cysteinate is present in amount of no greater than 10%, more preferably no greater than 5%, even more preferably no greater than 3%, still even more preferably no greater than 1%, and most preferably no greater than 0.5% by weight of the composition. Preferably, the lysine carboxymethyl cysteinate is present in amount of at least 0.00001%, more preferably at least 0.0001%, even more preferably at least 0.001%, still even more preferably at least 0.01%, and most preferably at least 0.1% by weight of the composition.

The composition of the present invention comprises a hydroxyl-substituted fatty acid. Fatty acid as used herein refers to carboxylic acids with an aliphatic chain of at least 6 carbon atoms, preferably 6 to 30 carbon atoms. The fatty acid can be branched or unbranched, preferably unbranched fatty acids. Preferably, the hydroxyl-substituted fatty acid comprises a straight chain having at least 4, more preferably at least 6 carbon atoms, even more preferably a straight chain having from 8 to 30 carbon atoms, and still even more preferably from 10 to 26 carbon atoms and most preferably from 12 to 24 carbon atoms.

Preferably, the hydroxyl-substituted fatty acid is hydroxyl-substituted C₈-C₂₄ fatty acid, more preferably hydroxyl-substituted C₁₀-C₂₂ fatty acid, even more preferably hydroxyl-substituted C₁₂-C₂₀ fatty acid, and most preferably hydroxyl-substituted C₁₆-C₂₀ fatty acid. Preferably, the hydroxyl-substituted fatty acid comprises hydroxy myristic acid, hydroxy palmitic acid, hydroxystearic acid, or a mixture thereof. More preferably, the hydroxyl-substituted fatty acid comprises hydroxystearic acid. Even more preferably, the hydroxyl-substituted fatty acid comprises comprises 12-hydroxystearic acid and most preferably the hydroxyl-substituted fatty acid is 12-hydroxystearic acid (12HSA). For sake of clarity, the hydroxyl-substituted fatty acid also includes salt of the hydroxyl-substituted fatty acid, especially sodium salt thereof but preferably, the hydroxyl-substituted fatty acid is free hydroxyl-substituted fatty acid.

The amount of hydroxyl-substituted fatty acid in the composition is preferably from 0.0001 to 7% by weight of the composition, more preferably from 0.001 to 5%, even more preferably from 0.005 to 3%, still even more preferably from 0.01 to 1% and most preferably from 0.05 to 0.6% by weight of the composition. The amount of hydroxyl-substituted C₈-C₂₄ fatty acid in the composition is preferably from 0.0001 to 7% by weight of the composition, more preferably from 0.001 to 5%, even more preferably from 0.005 to 3%, still even more preferably from 0.01 to 1% and most preferably from 0.05 to 0.6% by weight of the composition. The amount of 12-hydroxystearic acid in the composition is preferably from 0.0001 to 7% by weight of the composition, more preferably from 0.001 to 5%, even more preferably from 0.005 to 3%, still even more preferably from 0.01 to 1% and most preferably from 0.05 to 0.6% by weight of the composition.

Preferably, the molar ratio of the carboxymethyl cysteine compound to the hydroxyl substituted fatty acid is 3:1 to 100:1, more preferably 1:1 to 25:1, even more preferably 2:1 to 15:1 and still even more preferably 3:1 to 8:1. Preferably, the molar ratio of the lysine carboxymethyl cysteinate to the hydroxyl substituted fatty acid is 3:1 to 100:1, more preferably 1:1 to 25:1, even more preferably 2:1 to 15:1 and still even more preferably 3:1 to 8:1. Preferably, the molar ratio of the lysine carboxymethyl cysteinate to the 12-hydroxystearic acid is 3:1 to 100:1, more preferably 1:1 to 25:1, even more preferably 2:1 to 15:1 and still even more preferably 3:1 to 8:1.

Preferably, the composition comprises Vitamin B3 compounds (including derivatives of vitamin B3). The vitamin B3 compounds comprises niacin, nicotinic acid, niacinamide or a mixture thereof. The most preferred vitamin B3 compound is niacinamide. Amount of Vitamin B3 compounds may be 0.1 to 10%, preferably 0.5 to 5% by weight of the composition.

Preferably, the composition comprises a glutamate source selected from the group consisting of glutamine, glutamine ester, glutamic acid, pyroglutamic acid, salts, and mixtures thereof. More preferably, the composition comprises pyroglutamic acid and/or salt of pyroglutamic acid. Even more preferably, the composition comprises sodium salt of pyroglutamic acid. Preferably, the glutamate source is present in amount of 0.0001 to 10% by weight of the composition, more preferably 0.001 to 6%, even more preferably 0.01 to 3% by weight of the composition.

The composition may optionally comprise whitening pigment. Whitening pigments are typically particles of high refractive index materials. For example, the whitening pigment may have a refractive index of greater than 1.3, more preferably greater than 1.8 and most preferably from 2.0 to 2.7. Examples of such whitening pigment are those comprising bismuth oxy-chloride, boron nitride, barium sulfate, mica, silica, titanium dioxide, zirconium oxide, aluminium oxide, zinc oxide or combinations thereof. More preferred whitening pigment are particles comprising titanium dioxide, zinc oxide, zirconium oxide, mica, iron oxide or a combination thereof. Even more preferred whitening pigment are particles comprising zinc oxide, zirconium oxide, titanium dioxide or a combination thereof as these materials have especially high refractive index. Still even more preferably the whitening pigment is selected from titanium dioxide, zinc oxide or a mixture thereof and most preferred whitening pigment is titanium dioxide. The average diameter of whitening pigment is typical from 15 nm to 1 micron, more preferably from 35 nm to 800 nm, even more preferably from 50 nm to 500 nm and still even more preferably from 100 to 300 nm. Amount of whitening pigment may be 0.1 to 15%, preferably 0.5 to 5% by weight of the composition.

Preferably, the composition comprises polyhydric alcohol. Polyhydric alcohols may be selected from group of glycerin, propylyene glycol, dipropylene glycol, polypropylene glycol, polyethylene glycol, sorbitol, hydroxypropyl sorbitol, hexylene glycol, 1,3-butylene glycol, isoprene glycol, ethoxylated glycerol, propoxylated glycerol or a mixture thereof. Most preferred polyhydric alcohol is glycerol known also as glycerin. The amount of polyhydric alcohol may range anywhere from 0.1 to 20%, preferably 0.5 to 15% and more preferably 2 and 10% by weight of the composition.

Preferably, the composition comprises emollient materials. Suitable emollient materials include silicones, hydrocarbons, triglycerides or a mixture thereof. These silicones may be organic, silicone-containing or fluorine-containing, volatile or non-volatile, polar or non-polar. Hydrocarbons may include mineral oil, petrolatum and polyalpha-olefins. Examples of preferred volatile hydrocarbons include polydecanes such as isododecane and isodecane (e.g. Permethyl-99A which is available from Presperse Inc.) and the C7-C8 through C12-C15 isoparaffins (such as the Isopar Series available from Exxon Chemicals). Illustrative triglycerides but not limiting are sunflower seed oil, cotton oil, canola oil, soybean oil, castor oil, borage oil, olive oil, shea butter, jojoba oil and mixtures thereof. Mono- and di- glycerides may also be useful. Particularly preferable are glyceryl monostearate and glyceryl distearate.

Preferably, the composition comprises moisturizing agents. Particularly preferred moisturizing agents includes, petrolatum, aquaporin manipulating actives, oat kernel flour, substituted urea like hydroxyethyl urea, hyaluronic acid and/or its precursor N-acetyl glucosamine, hyaluronic acid and/or its precursor N-acetyl glucosamine, or a mixture thereof.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

The composition may comprise water in amount of 10 to 96% by weight of the composition, more preferably from 25 to 92%, even more preferably from 42 to 88%, most preferably from 55 to 82% by weight of the composition.

Preferably, the composition has a viscosity of at least 10 mPa·s, more preferably in the range 30 to 10000 mPa·s, even more preferably 50 to 5000 mPa·s, and most preferably 100 to 2000 mPa·s, when measured at 20 degrees C at a relatively high shear rate of about 20 s⁻¹. Preferably, the composition is in the form of fluid.

Preferably, the composition is an emulsion, more preferably an oil-in-water emulsion. The skin care composition refers to a composition suitable for topical application to human skin, including leave-on and wash-off products but preferably leave-on compositions. The term "leave-on" as used with reference to compositions herein means a composition that is applied to or rubbed on the skin, and left thereon. The term "wash-off" as used with reference to compositions herein means a skin cleanser that is applied to or rubbed on the skin and rinsed off substantially immediately subsequent to application. Preferably the skin care composition means a fluid liquid, and particularly a moisturizer rather than a make-up product.

Preferably, the personal care composition is a skin care composition. The term "skin" as used herein includes the skin on the face, neck, chest, abdomen, back, arms, under arms, hands, and legs. Preferably "skin" means includes the skin on the face and under arms, more preferably skin means skin on the face other than lips and eyelids.

Preferably the use is non-therapeutic. Preferably the method is non-therapeutic. The term non-therapeutic typically means for cosmetic purposes and not curative or therapeutic purposes.

The following examples are provided to facilitate an understanding of the invention. The examples are not intended to limit the scope of the claims.

### Examples

### Materials

| Supplier | Trade name | Active | Abbreviation | Active wt% |
|---|---|---|---|---|
| Sinerga | HAIR APP | Lysine carboxymethyl cysteinate | LCC | >98% |
| Sigma Aldrich | - | 12-hydroxystearic acid | 12-HSA | >98% |

### Example 1

This Example demonstrates the improved skin brightening efficacy by combining 12-hydroxystearic acid with lysine carboxymethyl cysteinate.

The skin brightening efficacy were evaluated by using MelaKuits^{®} living skin equivalent models (Biocell, Xi'an, China). A series of identical living skin equivalent models were constructed by following manufacturer's instruction. After the models were constructed, the models were irradiated by UVB with an energy density of 50 mJ/cm², followed by culturing in fresh medium with or without actives for one day. Such irradiating and culturing process were repeated by using refreshed same medium for seven times. The lightness (L*) of each model were measured by using a DSM II Color Meter. The measurements of lightness were repeated at least three times.

The results were summarised in Table 1.

**Table 1**

| Models | Irradiated by | Cultured by ^{#} | Average lightness (L*) |
|---|---|---|---|
| 1(Blank control) | - | medium | 43.4 |
| 2 | UVB | medium | 32.4 |
| 3 | UVB | 10 µM of 12-HSA in medium | 35.2 |
| 4 | UVB | 10 µM of 12-HSA + 50 µM of LCC in medium | 37.6 ^{a} |

| | | | |
|---|---|---|---|
| #: the concentration is based on the medium. a: Significant different with 3 (p<0.05). | | | |

It was surprisingly found by combining 12-hydroxystearic acid with Lysine carboxymethyl cysteinate, the skin brightening efficacy was significantly improved.

### Example 2

This Example demonstrates that lysine carboxymethyl cysteinate is not capable of improving the skin brightening efficacy of niacinamide.

Similar experiments were conducted as that in Example 1 except that niacinamide was used and different batch of live equivalent models were used. The original lightness of the live equivalent models was different from that in Example 1. The test results were summarised in Table 2.

**Table 2**

| Models | Irradiated by | Cultured by ^{#} | Average lightness (L*) |
|---|---|---|---|
| 5(Blank control) | - | medium | 71.43 |
| 6 | UVB | medium | 66.86 |
| 7 | UVB | 3 wt% of niacinamide in medium | 71.15 |
| 8 | UVB | 3 wt% of niacinamide and 0.27 wt% of LCC in medium | 72.44 ^{b} |

| | | | |
|---|---|---|---|
| #: the concentration is based on the medium. b: Not significant different with 7 (p<0.05). | | | |

It was shown in Table 2 that lysine carboxymethyl cysteinate is not capable of improving the skin brightening efficacy of niacinamide.

## Claims

1. A personal care composition comprising carboxymethyl cysteine compound and hydroxyl substituted fatty acid.

2. The composition according to claim 1 wherein the carboxymethyl cysteine compound comprises carboxymethyl cysteine, ester of carboxymethyl cysteine, and/or salt of carboxymethyl cysteine.

3. The composition according to claim 2 wherein the carboxymethyl cysteine compound comprises salt of carboxymethyl cysteine and preferably the carboxymethyl cysteine compound comprises lysine carboxymethyl cysteinate.

4. The composition according to any one of the preceding claims wherein the carboxymethyl cysteine compound is present in amount of at least 0.00001% and no greater than 10% by weight of the composition, preferably carboxymethyl cysteine compound is present in amount of at least 0.01% and no greater than 3% by weight of the composition.

5. The composition according to any one of the preceding claims wherein the hydroxyl-substituted fatty acid comprises a straight chain having from 8 to 30 carbon atoms, preferably from 12 to 24 carbon atoms.

6. The composition according to any one of the preceding claims wherein the hydroxyl-substituted fatty acid is hydroxyl-substituted C₈-C₂₄ fatty acid, the hydroxyl-substituted fatty acid is hydroxyl-substituted C₁₂-C₂₀ fatty acid.

7. The composition according to claim 6 wherein the hydroxyl-substituted fatty acid comprises hydroxy myristic acid, hydroxy palmitic acid, hydroxystearic acid, or a mixture thereof, preferably, the hydroxyl-substituted fatty acid comprises 12-hydroxystearic acid.

8. The composition according to any one of the preceding claims wherein the amount of hydroxyl-substituted fatty acid in the composition is preferably from 0.0001 to 7% by weight of the composition, preferably from 0.005 to 3%, by weight of the composition.

## Patentansprüche

1. Körperpflegezusammensetzung, umfassend eine Carboxymethylcysteinverbindung und eine Hydroxyl-substituierte Fettsäure.

2. Zusammensetzung nach Anspruch 1, wobei die Carboxymethylcysteinverbindung Carboxymethylcystein, Ester des Carboxymethylcysteins und/oder Salz des Carboxymethylcysteins umfasst.

3. Zusammensetzung nach Anspruch 2, wobei die Carboxymethylcysteinverbindung Salz des Carboxymethylcysteins umfasst und die Carboxymethylcysteinverbindung bevorzugt Lysincarboxymethylcysteinat umfasst.

4. Zusammensetzung nach irgendeinem der vorhergehenden Ansprüche, wobei die Carboxymethylcysteinverbindung in einer Menge von mindestens 0,00001 Gewichts-% und in nicht mehr als 10 Gewichts-% der Zusammensetzung vorhanden ist, wobei die Carboxymethylcysteinverbindung bevorzugt in einer Menge von mindestens 0,01 Gewichts-% und nicht mehr als 3 Gewichts-% der Zusammensetzung vorhanden ist.

5. Zusammensetzung nach irgendeinem der vorhergehenden Ansprüche, wobei die Hydroxyl-substituierte Fettsäure eine gerade Kette mit 8 bis 30 Kohlenstoffatomen, bevorzugt mit 12 bis 24 Kohlenstoffatomen, umfasst.

6. Zusammensetzung nach irgendeinem der vorhergehenden Ansprüche, wobei die Hydroxyl-substituierte Fettsäure Hydroxyl-substituierte C₈-C₂₄-Fettsäure und die Hydroxyl-substituierte Fettsäure Hydroxyl-substituierte C₁₂-C₂₀-Fettsäure ist.

7. Zusammensetzung nach Anspruch 6, wobei die Hydroxy-substituierte Fettsäure Hydroxymyristinsäure, Hydroxypalmitinsäure, Hydroxystearinsäure oder eine Mischung davon umfasst, wobei die Hydroxyl-substituierte Fettsäure bevorzugt 12-Hydroxystearinsäure umfasst.

8. Zusammensetzung nach irgendeinem der vorhergehenden Ansprüche, wobei die Menge der Hydroxyl-substituierten Fettsäure in der Zusammensetzung bevorzugt 0,0001 bis 7 Gewichts-% der Zusammensetzung beträgt, bevorzugt 0,005 bis 3 Gewichts-% der Zusammensetzung.

## Revendications

1. Composition de soins personnels comprenant un composé de carboxyméthylcystéine et un acide gras hydroxyl-substitué.

2. Composition selon la revendication 1, dans laquelle le composé de carboxyméthylcystéine comprend de la carboxyméthylcystéine, un ester de carboxyméthylcystéine et/ou un sel de carboxyméthylcystéine.

3. Composition selon la revendication 2, dans laquelle le composé de carboxyméthylcystéine comprend un sel de carboxyméthylcystéine et de préférence le composé de carboxyméthylcystéine comprend du carboxyméthylcystéinate de lysine.

4. Composition selon l'une quelconque des revendications précédentes, dans laquelle le composé de carboxyméthylcystéine est présent en une quantité d'au moins 0,00001 % et d'au plus 10 % en poids de la composition, de préférence le composé de carboxyméthylcystéine est présent en une quantité d'au moins 0,01 % et d'au plus 3 % en poids de la composition.

5. Composition selon l'une quelconque des revendications précédentes, dans laquelle l'acide gras hydroxyl-substitué comprend une chaîne droite ayant de 8 à 30 atomes de carbone, de préférence de 12 à 24 atomes de carbone.

6. Composition selon l'une quelconque des revendications précédentes, dans laquelle l'acide gras hydroxyl-substitué est un acide gras en C₈-C₂₄ hydroxyl-substitué, l'acide gras hydroxyl-substitué est un acide gras en C₁₂-C₂₀ hydroxyl-substitué.

7. Composition selon la revendication 6, dans laquelle l'acide gras hydroxyl-substitué comprend l'acide hydroxymyristique, l'acide hydroxypalmitique, l'acide hydroxystéarique ou un mélange de ceux-ci, de préférence l'acide gras hydroxyl-substitué comprend l'acide 12-hydroxystéarique.

8. Composition selon l'une quelconque des revendications précédentes, dans laquelle la quantité d'acide gras hydroxyl-substitué dans la composition est de préférence de 0,0001 à 7 % en poids de la composition, de préférence de 0,005 à 3 % en poids de la composition.
